# EUROPÄISCHE PATENTSCHRIFT

(11) **EP 1 658 805 B1**
(45) Veröffentlichungstag und Bekanntmachung des Hinweises auf die Patenterteilung: **05.08.2009**
(21) Anmeldenummer: 05024491.2
(22) Anmeldetag: 10.11.2005
(51) Int. Cl.: A61B 1/005

(54) **Flexibler Schaft für ein Endoskop sowie derartiges Endoskop**
Flexible shaft for an endoscope and corresponding endoscope
Tige flexible pour un endoscope et endoscope correspondant

(30) Priorität: 19.11.2004 DE 102004057481
(43) Veröffentlichungstag der Anmeldung: 24.05.2006
(73) Patentinhaber: Karl Storz GmbH & Co. KG, 78532 Tuttlingen (DE)
(72) Erfinder: Wimmer, Viktor, 83370 Seeon (DE)
(74) Vertreter: Heuckeroth, Volker

(56) Entgegenhaltungen:
- WO-A-96/09009
- US-A- 4 329 980
- US-A- 4 669 172
- US-A- 4 977 887
- US-A1- 2002 002 323

## Beschreibung

Die Erfindung betrifft einen flexiblen Schaft für ein Endoskop, mit einem Schaftkörper, der in Längsrichtung des Schafts einen ersten Abschnitt mit einem ersten Flexibilitätsgrad und proximal von dem ersten Abschnitt zumindest einen zweiten Abschnitt mit einem zweiten Flexibilitätsgrad aufweist, der geringer ist als der erste Flexibilitätsgrad, wobei der Schaftkörper in dem ersten Abschnitt zumindest ein erstes Federelement und in dem zweiten Abschnitt zumindest ein zweites Federelement aufweist, wobei zumindest das zweite Federelement aus schraubenförmigen Windungen aufgebaut ist, wobei eine Federhärte des zweiten Federelements durch eine Vorspannung des zweiten Federelements erhöht ist, wodurch die Flexibilität des Schaftkörpers in dem zweiten Abschnitt verringert ist, wobei die Vorspannung durch eine axiale Stauchung der Windungen des zweiten Federelements relativ zueinander aufgebracht ist.

Die Erfindung betrifft ferner ein Endoskop, dass einen flexiblen Schaft der vorstehend genannten Art aufweist.

Ein Schaft der eingangs genannten Art sowie ein Endoskop mit einem solchen Schaft sind aus dem Dokument US-A-4 329 980 bekannt.

Ein Endoskop, dass einen flexiblen Schaft aufweist, wird auch als flexibles Endoskop bezeichnet. Derartige flexible Endoskope werden vor allem für medizinische Anwendungen eingesetzt, wobei die vorliegende Erfindung jedoch auch bei flexiblen Endoskopen verwendet werden kann, die für technische Zwecke, beispielsweise zur Inspektion von Maschinen, eingesetzt werden können.

Flexible Endoskope unterscheiden sich von starren Endoskopen dadurch, dass der Schaft bei einem flexiblen Endoskop eine solche Flexibilität aufweist, dass er eine gebogene, eine stark gekrümmte oder sogar eine schlaufenförmige Form einnehmen kann.

Solche flexiblen Endoskope eignen sich daher insbesondere als medizinische Operations- oder Untersuchungsendoskope für solche inneren Körperpartien, die verzweigte oder verschlungene Strukturen aufweisen, wie beispielsweise der Magen- und Darmbereich oder die Atemwege einschließlich der Lunge.

Ein zur Untersuchung des Dickdarms verwendetes flexibles Endoskop, gegebenenfalls mit der Möglichkeit zur Biopsie und zur Durchführung kleiner operativer Eingriffe wird auch als Koloskop bezeichnet.

Ein flexibles Endoskop hat weiterhin die Eigenschaft, dass der distale Endabschnitt des flexiblen Schafts eine noch weiter erhöhte Beweglichkeit bzw. Flexibilität dahingehend aufweist, dass dieser distale Endabschnitt, der auch als "Deflecting" bezeichnet wird, gegenüber dem übrigen Teil des Schafts aus dessen Längsrichtung mittels einer Steuereinrichtung ausgelenkt, d. h. abgebogen werden kann. In der Regel lässt sich der distale Endabschnitt in einer Ebene zu beiden Seiten der Geradeausstellung beispielsweise in einem Winkelbereich von mehr als ±90° auslenken. Die zum Auslenken des distalen Endabschnitts vorgesehene Steuereinrichtung ist am proximalen Ende des flexiblen Schafts angeordnet, die üblicherweise ein manuell bedienbares Betätigungselement umfasst, das über einen Zugseilmechanismus mit dem distalen Endabschnitt in Wirkverbindung steht, so dass der Operateur die Auslenkung des distalen Endabschnitts den Erfordernissen entsprechend einstellen und verändern kann. Die Auslenkung des distalen Endabschnitts ermöglicht die Einstellung einer Vielzahl von Blickrichtungen durch das Endoskop.

Während ein hoher Flexibilitätsgrad der Schaftfeder eines flexiblen Endoskops im auslenkbaren distalen Endabschnitt gewünscht ist, ist im proximalen Bereich des Endoskopschafts ein geringerer Flexibilitätsgrad d. h. eine höhere Steifigkeit des flexiblen Schafts erwünscht. Dies liegt darin begründet, dass der flexible Schaft insgesamt eine erhebliche Länge aufweisen kann. Beim Vorschieben des Endoskopschafts, beispielsweise im Darm eines Patienten, sollte die Schubkraft nicht zu einem Verkrümmen des Schafts im proximalen Bereich führen. Wenn sich der Schaft beim Einführen in den Darm verkrümmt bzw. gar im und mit dem Darm verkrümmt, kommt es zu einer schmerzhaften Schleifenbildung des Darms. Mit anderen Worten sollte der Schaft des Endoskops beim Vorschieben im proximalen Bereich möglichst gerade bleiben, was im proximalen Bereich trotz gewährleisteter Flexibilität eine gewisse Stabilität oder Tendenz des Schafts erfordert, sich bei einer Verkrümmung selbstständig wieder zu begradigen.

Der aus dem Dokument US-A-6 485 411 bekannte flexible Schaft für ein Endoskop weist einen Schaftkörper auf, der aus einer Mehrzahl an einstückig miteinander verbundenen Federelementen aufgebaut ist, die in der Art von Schraubenfedern ausgebildet ist. Die einzelnen Wendeln der Federelemente bestehen aus einem bandförmigen Flachmaterial, wobei die Wendeln in Längsrichtung des Schafts eine bestimmte Breite aufweisen.

Der Schaftkörper dieses bekannten flexiblen Schafts weist zumindest drei Abschnitte unterschiedlicher Flexibilitätsgrade auf. Ein distaler Abschnitt weist dabei den höchsten Flexibilitätsgrad auf, d. h. ist am flexibelsten, der sich daran anschließende nächste Abschnitt weist einen demgegenüber geringeren Flexibilitätsgrad und der sich daran proximal anschließende Abschnitt einen noch geringeren Flexibilitätsgrad auf. Dieser bekannte Schaft weist demnach eine von distal nach proximal abgestuft abnehmende Flexibilität auf.

Die verschiedenen Flexibilitätsgrade bzw. - stufen werden bei diesem bekannten Schaft dadurch erreicht, dass die Breite der einzelnen Wendeln der Federelemente von distal nach proximal zunehmen, wodurch der Flexibilitätsgrad von distal nach proximal abnimmt. Es ist dort beschrieben, dass jedoch auch die unterschiedlichen Flexibilitätsgrade bei gleichen Breiten der Wendeln in Längsrichtung des Schafts durch unterschiedliche Beabstandungen benachbarter Wendeln der einzelnen Federelemente erreicht werden, die bei der Herstellung der Federelemente durch die axiale Breite der Materialwegnahme zwischen den Wendeln eingeteilt werden.

Es hat sich bei einem so aufgebauten Schaftkörper gezeigt, dass dieser im Laufe der Zeit nach einer Vielzahl von Anwendungen Ermüdungserscheinungen zeigt, die in einem erhöhten und unerwünschten Spiel der Steuereinrichtung zum Auslenken des distalen Endabschnitts des Schafts resultieren. Dadurch wird eine genaue Positionierung des Endoskops im Untersuchungs- bzw. Behandlungsareal im menschlichen Körper erschwert.

Des weiteren hat sich gezeigt, dass ein solcher flexibler Schaft insbesondere im proximalen Bereich durch die Lastwechsel beim Einführen und Herausziehen des Endoskops seine Stabilität insbesondere im proximalen Bereich verliert, d. h. die Flexibilität dort zunimmt. Dies wiederum führt unerwünschterweise dazu, dass die beim Einführen aufgebrachte Schubkraft zu einem Verkrümmen bzw. Ausknicken des Schafts im proximalen Bereich führt, weil die Tendenz des Schafts, sich selbst zu begradigen, im Laufe der Zeit durch Ermüdung der Federelemente nachlässt. Diese Ermüdung des Schaftkörpers im proximalen Bereich wirkt sich auch auf den den Schaftkörper außen umgebenden Außenmantel, der üblicherweise aus einem Kunststoffschlauch besteht, dahingehend aus, dass dieser zur Falten- und Rissbildung neigt.

Demgegenüber weist der aus dem eingangs genannten Dokument US-A 4 329 980 bekannte flexible Schaft ein flexibles zylindrisches Hohlrohr und eine einzelne Schraubenfeder auf, von der ein Ende mit einem Ende des zylindrischen Hohlrohrs in Eingriff bringbar ist, wodurch die Flexibilität des flexiblen Schafts eingestellt werden kann. Dazu ist ein Mechanismus zum Zusammendrücken der Schraubenfeder am anderen Ende des zylindrischen Hohlrohrs vorgesehen, der einen Schieber aufweist, der mit dem anderen Ende der einzelnen Schraubenfeder in Eingriff steht, sowie einen Betätigungsabschnitt zum Hin- und Herbewegen des Elements zum Zusammendrücken der Feder zwischen einer Stellung, in der das Element zum Zusammendrücken der Feder die einzelne Schraubenfeder zusammendrückt und einer Stellung, in der das Element zum Zusammendrücken der Feder axial von dem Ende der Schraubenfeder entfernt ist. Bei diesem flexiblen Schaft ist demnach die axiale Stauchung der Windungen der Schraubenfeder einstellbar.

Nachteilig an diesem bekannten flexiblen Schaft ist die Komplexität und damit kostenaufwändige Ausgestaltung des Schieberantriebs zum Komprimieren der Schraubenfeder.

Ferner ist aus dem Dokument DE 697 23 193 C2 eine Behandlungszange zur Verwendung mit einem Endoskop bekannt, die einen flexiblen Schaft aus einer enggewickelten Edelstahlspirale aufweist. Der Schaft weist einen Abschnitt auf, der leichter biegbar ist als der übrige Abschnitt. Dieser unterschiedliche Flexibilitätsgrad des Schafts wird durch eine Vorspannung des einen Abschnitts der Edelstahlspirale erreicht, wobei die Vorspannung derart ist, dass sich benachbarte Windungen der Spirale einander berühren, um die Steifheit zu erhöhen.

Der Erfindung liegt die Aufgabe zu Grunde, einen flexiblen Schaft für ein Endoskop der eingangs genannten Art dahingehend weiterzubilden, dass die vorstehend genannten Nachteile vermieden werden, dass insbesondere die Flexibilität des Schaftkörpers in dem zweiten Abschnitt auf konstruktiv einfache Weise verringert ist.

Erfindungsgemäß wird diese Aufgabe hinsichtlich des eingangs genannten flexiblen Schafts für ein Endoskop dadurch gelöst, dass die Vorspannung permanent ist und durch ein flexibles Zugelement aufgebracht wird, das das zweite Federelement axial zusammenzieht, wobei das Zugelement an zwei axial beabstandeten Stellen an dem zweiten Federelement befestigt ist, deren Abstand der Länge des zweiten Federelements im komprimierten Zustand entspricht.

Bei dem erfindungsgemäßen flexiblen Schaft wird die Verringerung der Flexibilität des Schaftkörpers in dem zweiten Abschnitt durch eine permanente Vorspannung des zweiten Federelements bewirkt. Das zweite Federelement steht somit unter einer permanenten Vorspannung, die eine Stabilisierung des Schaftes in Richtung seiner Längsachse bewirkt, d. h. die Tendenz des Schafts im zweiten Abschnitt, sich einer Verbiegung zu Wiedersetzen bzw. sich nach einer Auslenkung wieder selbst zu Begradigen, ist durch die permanente Vorspannung des zweiten Federelements erhöht. Diese Ausgestaltung des Schaftkörpers zumindest im zweiten Abschnitt geht also nicht von der bekannten Maßnahme aus, die Federhärte des Federelements durch eine Zunahme der Breite der Wendeln in axialer Richtung zu erhöhen, sondern durch eine Vorspannung, wodurch der Schaft gegenüber Lastwechseln, die auf das Federelement wirken, weniger ermüdungsanfällig ist.

Dabei ist zumindest das zweite Federelement aus schraubenförmigen Windungen aufgebaut, und die Vorspannung ist durch eine axiale Stauchung der Windungen relativ zueinander aufgebracht. Bei dieser Ausgestaltung ist zumindest das zweite Federelement wie der Schaftkörper des bekannten Schafts aus einer Schraubenfeder aufgebaut, wobei jedoch die höhere Federhärte des zweiten Federelements nicht durch eine Verbreiterung der Windungen bzw. Wendeln in axialer Richtung erreicht wird, sondern durch eine Kompression der Schraubenfeder. Dies hat auch den Vorteil einer einfacheren Herstellbarkeit unterschiedlicher Federhärten, da der erste und der zweite Abschnitt beispielsweise aus insgesamt einem Federelement gebildet sein können und vorzugsweise auch ausgebildet sind, wobei der den zweiten Abschnitt des Schaftkörpers bildende Abschnitt des Federelements permanent komprimiert ist. Ein Schaftkörper mit zumindest zwei Abschnitten unterschiedlicher Flexibilitätsgrade ist auf diese Weise besonders leicht herstellbar, ohne dass zwei Federelemente mit unterschiedlichen Geometrien der Wendeln oder Windungen verwendet werden müssen. Die Kompression kann so stark sein, dass sich benachbarte Windungen berühren.

Erfindungsgemäß wird die Vorspannung durch ein flexibles Zugelement aufgebracht, das das zweite Federelement axial zusammenzieht.

Ein solches Zugelement, dass beispielsweise in Form eines oder mehrerer flexibler Zugdrähte oder Zugseile ausgebildet sein kann, hat den Vorteil, dass es mit dem zweiten Federelement leicht verbunden werden kann, beispielsweise durch eine bekannte Verbindungstechnik wie Schweißen, Löten oder Kleben.

Dabei ist es bevorzugt, wenn das Zugelement nur an zwei axial beabstandeten Stellen an dem zweiten Federelement befestigt ist, deren Abstand der Länge des zweiten Federelements im komprimierten Zustand entspricht.

Hierbei ist von Vorteil, dass die relative Beweglichkeit des Federelements zu dem Zugelement zwischen den beiden Befestigungsstellen des Zugelements am zweiten Federelements erhalten bleibt, was für die Funktion des zweiten Federelements bedeutsam ist, nämlich dass es sich in jede Richtung leicht Auslenken lässt, was für einen flexiblen Endoskopschaft wichtig ist.

In einer weiteren bevorzugten Ausgestaltung ist das Zugelement ein zumindest das zweite Federelement umgebendes netzartiges Hüllelement.

Ein solches netzartiges Hüllelement, dass beispielsweise aus einem Metallnetz besteht, hat des weiteren den Vorteil eines Schutzes des Federelements bzw. der Federelemente des Schaftkörpers. Des weiteren ermöglicht ein derartiges netzartiges Hüllelement insbesondere in Verbindung mit der zuvor genannten Ausgestaltung, wonach das Hüllelement an nur zwei axial beabstandeten Stellen an dem zweiten Federelement befestigt ist, dass das zweite Federelement entlang der Innenwand des netzartigen Hüllelements gleitend beweglich bleibt. Das Hüllelement trägt auch vorteilhafterweise zu einer Verwindungssteifigkeit des Schafts bei.

Bevorzugt ist es dabei weiterhin, wenn sich das Hüllelement über die gesamte Länge des Schaftkörpers erstreckt.

Hierbei ist ein Vorteil, dass die Außenkontur des flexiblen Schafts eben, d. h. frei von Vorsprüngen und Unebenheiten ausgebildet werden kann.

Die Verwendung eines netzartigen Hüllelements hat des weiteren den Vorteil, dass sich dieses besonders leicht auf den Federelementen aufziehen lässt, was die Herstellung des Schafts vorteilhafterweise weiterhin vereinfacht, und es müssen nur wenige Fixierstellen zum Fixieren des Hüllelements über die Länge des Schaftkörpers vorgesehen werden.

In einer weiteren bevorzugten Ausgestaltung ist das Hüllelement von einem Außenmantel umgeben, der mit dem Hüllelement fest verbunden ist.

Ein solcher Außenmantel, der vorzugsweise aus Kunststoff gefertigt ist, bewirkt in Verbindung mit dem Hüllelement vorteilhafterweise, dass der flexible Schaft insgesamt verwindungssteif ausgebildet werden kann, so dass Drehbewegungen des Schafts um seine Längsachse nicht zu Verwindungen des Schafts führen. Die Verbindung zwischen dem Außenmantel und dem Hüllelement ist vorzugsweise formschlüssig, indem beispielsweise der Außenmantel mit dem netzartigen Hüllelement unter Wärmeeinwirkung verbunden wird.

In einer weiteren bevorzugten Ausgestaltung sind das erste Federelement und das zumindest zweite Federelement einstückig miteinander ausgebildet.

Hierbei ist ein Vorteil, dass die Anzahl der für den Aufbau des Schaftkörpers benötigten Teile verringert ist. Insbesondere können das erste Federelement und das zumindest zweite Federelement als Abschnitte ein und desselben Federelements ausgebildet sein, wobei, wie oben bereits beschrieben, die Federhärte des zweiten Abschnitts dieses Federelements durch die erfindungsgemäß vorgesehene permanente Vorspannung gegenüber dem übrigen Teil des Federelements erhöht wird.

In einer weiteren bevorzugten Ausgestaltung weist der Schaftkörper zumindest einen dritten Abschnitt mit einem dritten Flexibilitätsgrad auf, wobei der Schaftkörper in dem dritten Abschnitt ein drittes Federelement aufweist, wobei der dritte Flexibilitätsgrad geringer als der erste Flexibilitätsgrad und geringer als der zweite Flexibilitätsgrad ist.

Bei dieser Ausgestaltung weist der flexible Schaft zumindest drei Abschnitte mit unterschiedlichen Flexibilitätsgraden auf, wobei der zumindest dritte Abschnitt mit dem zumindest dritten Federelement, da es die geringste Federhärte aufweist, vorzugsweise den distalen Endabschnitt des Schafts bildet. Wegen der erfindungsgemäß vorgesehenen Vorspannung des zweiten Federelements werden für die Erzielung dreier unterschiedlicher Flexibilitätsgrade jedoch im Unterschied zum Stand der Technik nur zwei Federelemente mit unterschiedlichen Geometrien benötigt, da der zweite Flexibilitätsgrad durch eine Vorspannung des zweiten Federelements erreicht wird.

In diesem Zusammenhang ist es wiederum bevorzugt, wenn das erste, zweite und zumindest dritte Federelement einstückig miteinander ausgebildet sind.

Bei dieser Ausgestaltung wird auch bei einem zumindest dreistufig flexiblen Schaft der Herstellungsaufwand vorteilhaft niedrig gehalten.

In einer weiteren bevorzugten Ausgestaltung weisen das erste Federelement und das zweite Federelement im nicht vorgespannten Zustand des zweiten Federelements die gleiche Federhärte auf.

Diese Maßnahme trägt vorteilhafterweise ebenfalls zur einfacheren Herstellung des erfindungsgemäßen Schafts bei, da das erste Federelement und das zweite Federelement als Abschnitte ein und desselben einteiligen Federelements zunächst bereitgestellt werden können, wobei dann ein Abschnitt dieses einteiligen Federelements mit einer Vorspannung beaufschlagt wird, wodurch dieser vorgespannte Abschnitt eine größere Federhärte erhält als der übrige Abschnitt des Federelements.

Die Erfindung betrifft ferner ein Endoskop, dass einen flexiblen Schaft gemäß einer oder mehreren der vorstehend genannten Ausgestaltungen aufweist.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Figur 1: ein Endoskop mit einem flexiblen Schaft in einer Gesamtdarstellung;
- Fig. 1A: ein distales Ende des Schafts des Endoskops in Figur 1;
- Figur 2: den Schaft des Endoskops in Figur 1 in vergrößertem Maßstab in Alleinstellung und in unterbrochener Darstellung, teilweise mit Aufbrechungen;
- Figur 3: den Schaft in Figur 2 unter Weglassung des Außenmantels;
- Figur 4: den Schaft in Figuren 2 und 3 unter weiterer Weglassung des netzartigen Hüllelements.

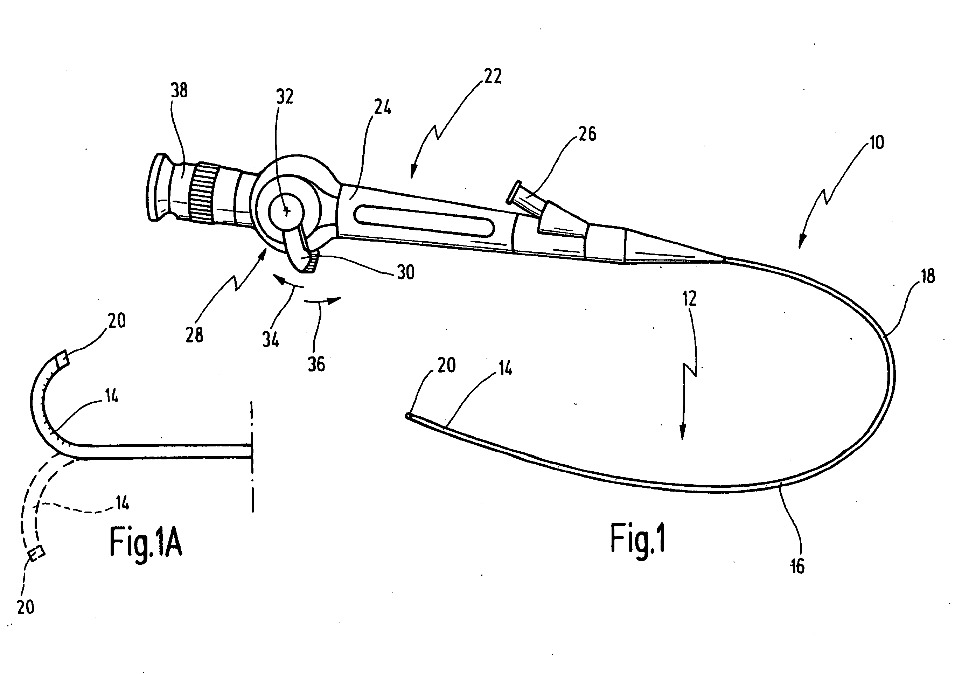

In Figur 1 ist ein insgesamt mit dem Bezugszeichen 10 versehenes flexibles Endoskop dargestellt. Das Endoskop 10 wird zu Untersuchungs- und/oder Operationszwecken in medizinischen Verfahren verwendet. Das Endoskop 10 ist beispielsweise ein Koloskop.

Das Endoskop 10 weist einen lang erstreckten, flexiblen Schaft 12 auf, der später noch in Einzelheiten beschrieben wird. In dem Schaft 12 verlaufen eine nicht dargestellte Endoskopoptik in Form von Lichtleitfasern, gegebenenfalls verschiedene Kanäle, wie ein Saug- und Spülkanal und gegebenenfalls ein Instrumentenkanal. Im Falle eines Koloskops ist der Schaft länger als bei einem Bronchoskop.

Der flexible Schaft 12 weist einen distalen Abschnitt 14, einen mittleren Abschnitt 16 und einen proximalen Abschnitt 18 auf. Das äußerste distale Ende des distalen Abschnitts 14 weist ein Endstück 20 in Form einer Abschlussbuchse auf.

Der Schaft 12 ist an seinem proximalen Ende mit einem Kopfstück 22 verbunden, das ein Gehäuse 24 aufweist.

Am distalen Ende des Kopfstücks 22 ist an dem Gehäuse 24 ein Anschluss 26 zum Einführen beispielsweise eines Instruments in den zuvor genannten Instrumentenkanal des Schafts 12 angeordnet.

Wie in Figur 1A dargestellt ist, ist der distale Abschnitt 14 des Schafts 12 aus der in Figur 1 dargestellten Geradeausstellung in zwei Richtungen auslenkbar, wobei eine Auslenkung des Abschnitts 14 aus der Geradeausstellung um nahezu 180° oder gar bis 210° in beiden Richtungen möglich ist. Bei einem Koloskop ist der distale Abschnitt 14 in vier Richtungen auslenkbar.

Zur Auslenkung des distalen Abschnitts 14 aus der Geradeausstellung ist ein nicht näher dargestellter Zugseilmechanismus vorgesehen, der mit einer Steuereinrichtung 28 am Kopfstück 22 in Wirkverbindung steht, so dass durch Betätigen der Steuereinrichtung 28 der distale Abschnitt 14 ausgelenkt werden kann.

Die Steuereinrichtung 28 ist hier eine manuell betätigbare Steuereinrichtung, die ein Betätigungselement 30 in Form eines Schwenkhebels aufweist, der um eine Drehachse 32 verschwenkbar ist. Wird das Betätigungselement 30 in Richtung eines Pfeils 34 um die Drehachse 32 verschwenkt, wird der distale Abschnitt 14 gemäß Figur 1A in der einen Richtung ausgelenkt bzw. abgekrümmt, wie mit durchgezogenen Linien in Figur 1A dargestellt ist, und durch Betätigen des Betätigungselements 30 in Richtung eines Pfeiles 36 wird der distale Abschnitt 14 in die entgegengesetzte Richtung ausgelenkt bzw. abgekrümmt, wie mit unterbrochenen Linien in Figur 1A dargestellt ist.

Am äußeren proximalen Ende des Kopfstücks 22 ist ferner ein Okular 38 angeordnet.

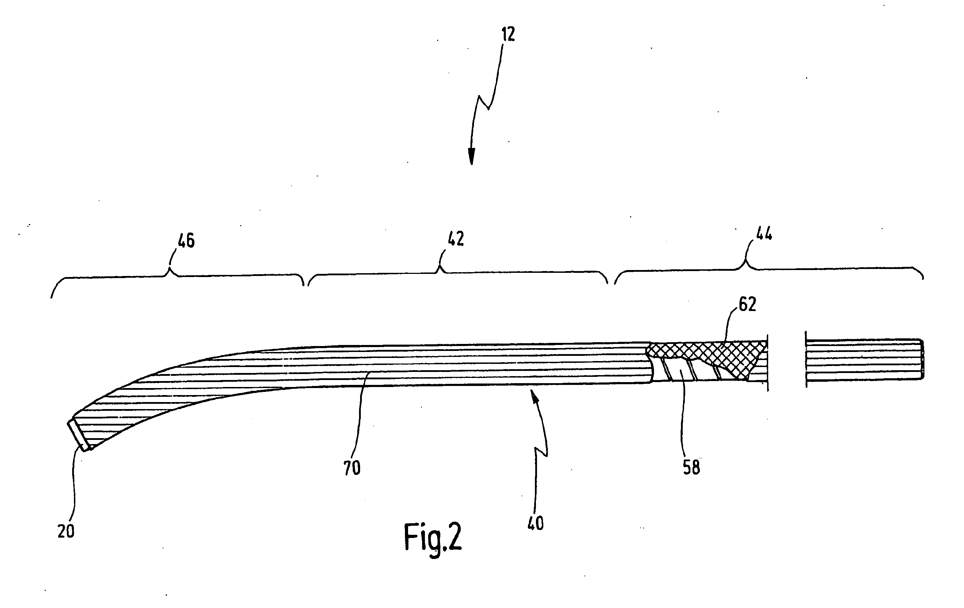

Nachfolgend wird mit Bezug auf Figuren 2 bis 4 die Ausgestaltung des Schafts 12 näher beschrieben.

Der Schaft 12 weist einen Schaftkörper 40 auf, der einen ersten Abschnitt 42, der dem mittleren Abschnitt 16 in Figur 1 entspricht, einen zweiten Abschnitt 44, der dem proximalen Abschnitt 18 in Figur 1 entspricht, und einem dritten Abschnitt 46 aufweist, der dem distalen Abschnitt 14 entspricht.

In den drei Abschnitten 42 bis 46 weist der Schaftkörper 40 jeweils einen unterschiedlichen Flexibilitätsgrad auf, wobei die Flexibilität des Schaftkörpers 40 im distalen dritten Abschnitt 46 am größten ist, d. h. in diesem Bereich ist der Schaftkörper 40 am flexibelsten.

In dem ersten Abschnitt 42, der in Längsrichtung proximal von dem dritten Abschnitt 46 angeordnet ist, ist die Flexibilität des Schaftkörpers 40 geringer als in dem dritten Abschnitt 46, und in dem zweiten Abschnitt 44, der sich proximal an den ersten Abschnitt 42 anschließt, ist die Flexibilität des Schaftkörpers 40 geringer als in dem Abschnitt 46 und auch geringer als in dem Abschnitt 42.

In jedem der Abschnitte 42, 44 und 46 weist der Schaftkörper 40 ein Federelement auf, und zwar in dem dritten Abschnitt 46 ein drittes Federelement 48, in dem ersten Abschnitt 42 ein erstes Federelement 50 und in dem zweiten Abschnitt 44 ein zweites Federelement 52.

Die Federelemente 48 bis 52 sind in Form von Schraubenfedern ausgebildet und weisen jeweils eine Mehrzahl von Schraubenförmigen Wendeln oder Windungen 54, 56 bzw. 58 auf.

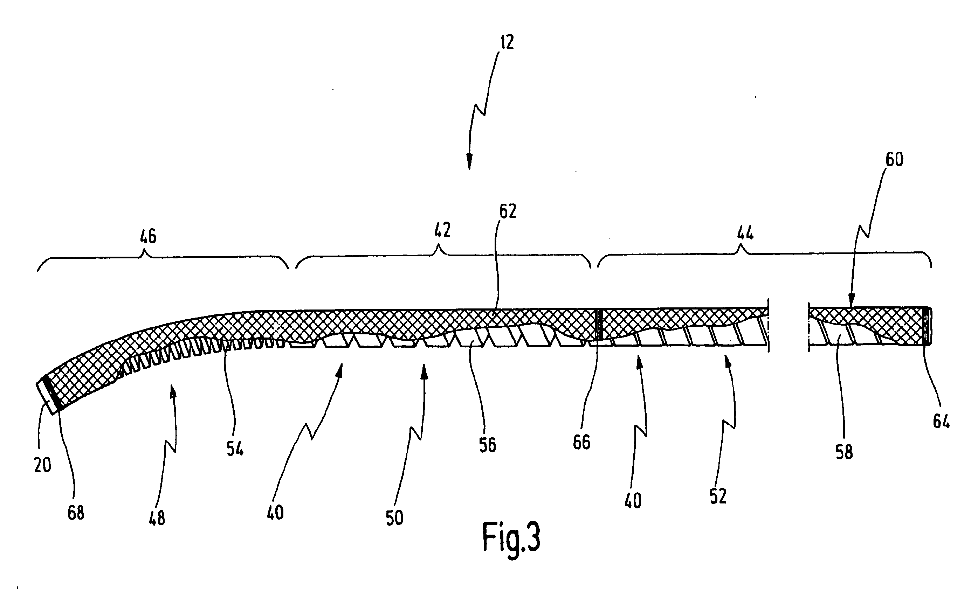

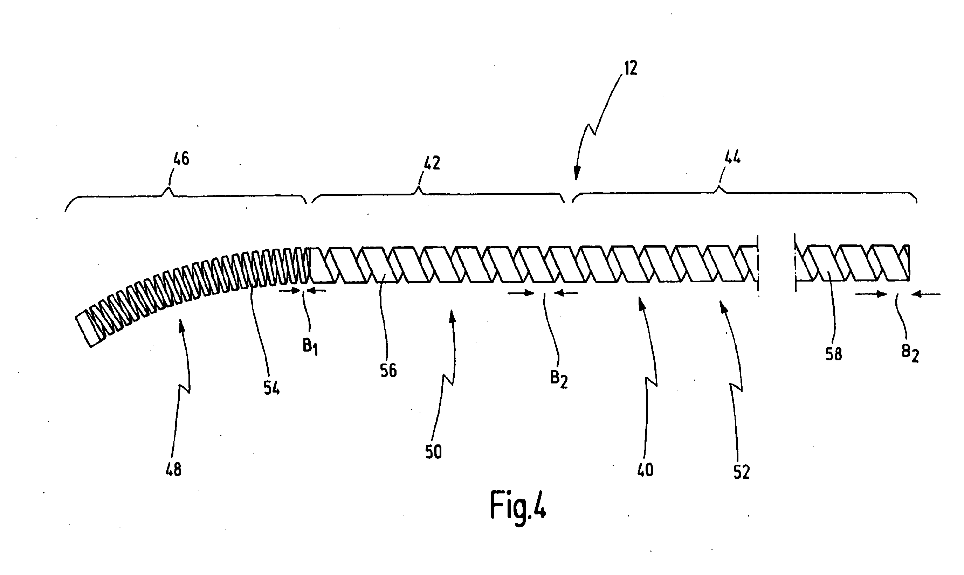

Wie aus Figuren 3 und 4 hervorgeht sind die Federelemente 48, 50 und 52 aus einem streifenförmigen oder bandförmigen Flachmaterial gebildet. Die Federelemente 48 bis 52 können aus einem rohrförmigen Material durch Laserschneiden gefertigt sein.

Eine Erstreckung oder Breite B₁ der einzelnen Windungen 54 des dritten Federelements 48 ist dabei geringer als eine Breite B₂ der Windungen 56 des ersten Federelements 50 und der Windungen 58 des zweiten Federelements 52. Die Breite B₂ der Windungen 56 des ersten Federelements 50 und der Windungen 58 des zweiten Federelements 52 sind dabei gleich.

Gemäß Figur 3 ist jedoch trotz gleicher Breiten B₂ der Windungen 56 und 58 des ersten Federelements 50 und des zweiten Federelements 52 die Federhärte des zweiten Federelements 52 gegenüber der Federhärte des ersten Federelements 50 erhöht, indem das zweite Federelement 52 gemäß Figur 3 permanent vorgespannt ist, indem das zweite Federelement 52, wie am Besten in Figur 3 erkennbar ist, axial gestaucht bzw. komprimiert ist. Durch diese durch Stauchung bzw. Kompression aufgebrachte permanente Vorspannung ist die Flexibilität im zweiten Abschnitt 44 des Schaftkörpers 40 gegenüber dem ersten Abschnitt 42 herabgesetzt, obwohl, wie in Figur 4 dargestellt ist, die Federelemente 50 und 52, wenn das zweite Federelement 52 nicht vorgespannt wäre, identisch sind.

Die Federelemente 48, 50 und 52 sind einstückig miteinander ausgebildet, wobei gemäß Figur 4 die Federelemente 50 und 52 sogar Abschnitte ein und desselben Federelements darstellen. Das Federelement 48 kann dagegen ein separates Federelement sein, dass mit dem ersten Federelement 50 an der Verbindungsstelle beispielsweise durch Laserschweißen fest verbunden ist.

Die permanente Vorspannung des zweiten Federelements 52 wird durch ein Zugelement 60 aufgebracht, dass das zweite Federelement 52 axial zusammenzieht.

Das Zugelement 60 ist in Form eines netzartigen Hüllelements 62 ausgebildet, das beispielsweise aus einem metallischen dünnen rohrförmigen Drahtnetz besteht. Das Hüllelement 62 erstreckt sich von dem distalen Endstück 20 bis zum proximalen Ende des Schaft 12. Das netzartige Hüllelement 62 liegt unmittelbar auf den Federelementen 48, 50 und 52 auf, ist an diesen jedoch nur an bestimmten Stellen fixiert, wie hiernach beschrieben wird. Das Hüllelement 62 ist insbesondere torsionsstabil.

Eine erste Befestigungsstelle 64 befindet sich am proximalen Ende des Schaftkörpers 40, und zwar am proximalen Ende des zweiten Federelements 52. Eine zweite Befestigungsstelle 66 befindet sich am distalen Ende des zweiten Federelements 52, wobei der Abstand der Befestigungsstellen 64 und 66 der Länge des zweiten Federelements 52 in dessen komprimierten Zustand gemäß Figur 3 entspricht. Durch die Befestigung des Hüllelements 62 an dem zweiten Federelement 52 an den Stellen 64 und 66 bringt das Hüllelement 62 entsprechend die Zugkraft zur permanenten Stauchung bzw. Kompression des zweiten Federelements 52 und damit die permanente Vorspannung des zweiten Federelements 52 auf. Zwischen den Stellen 64 und 66 ist das Hüllelement 62 nicht mit dem zweiten Federelement 52 verbunden, so dass die Windungen 58 des zweiten Federelements 52 eine relative Beweglichkeit zu dem Hüllelement 62 zwischen den Befestigungsstellen 64 und 66 besitzen.

Eine weitere Befestigungsstelle 68 befindet sich am distalen Ende des Schaftkörpers 40, und zwar am Endstück 20. Alle Befestigungsstellen 64, 66 und 68 sind vorzugsweise als Lötverbindungen zwischen dem metallischen Hüllelement 62 und den metallischen Federelementen bzw. dem metallischen Endstück 20 ausgebildet.

Zwischen den Befestigungsstellen 66 und 68 ist das Hüllelement 62 ebenfalls relativ zu den Windungen 54 und 56 der Federelemente 48 und 50 beweglich.

Bei der Herstellung des Schafts 12 wird der Schaftkörper 40 mit den Federelementen 48, 50 und 52 und dem Endstück 20 einstückig gemäß Figur 4 bereit gestellt, wobei in diesem Zustand das zweite Federelement 52 noch nicht vorgespannt ist. Anschließend wird das Hüllelement 62 beispielsweise vom Endstück 20 her über die Federelemente 48 bis 52 geschoben, wobei vor dem Fixieren des Hüllelements 62 am proximalen Ende des zweiten Federelements 52 dieses axial zusammengedrückt wird, und anschließend wird das Hüllelement 62 an der Befestigungsstelle 64 vollumfängliche an dem zweiten Federelement 52 befestigt.

Das Hüllelement 62 wiederum ist gemäß Figur 2 von einem Außenmantel 70 umgeben, der sich ebenfalls über die gesamte Länge des Schafts 12 und damit über die gesamte Länge des Hüllelements 62 erstreckt.

Der Außenmantel 70 besteht aus Kunststoff und ist durch Wärmeeinwirkung mit dem Hüllelement 62 verbunden, insbesondere verbacken, wodurch der Außenmantel 70 mit dem Hüllelement 62 formschlüssig verbunden ist und der Schaft 12 so eine höhere Torsionsstatikkeit besitzt.

## Patentansprüche

1. Flexibler Schaft für ein Endoskop (10), mit einem Schaftkörper (40), der in Längsrichtung des Schafts (12) einen ersten Abschnitt (42) mit einem ersten Flexibilitätsgrad und proximal von dem ersten Abschnitt (42) zumindest einen zweiten Abschnitt (44) mit einem zweiten Flexibilitätsgrad aufweist, der geringer ist als der erste Flexibilitätsgrad, wobei der Schaftkörper (40) in dem ersten Abschnitt (42) zumindest ein erstes Federelement (50) und in dem zweiten Abschnitt (44) zumindest ein zweites Federelement (52) aufweist, wobei zumindest das zweite Federelement (52) aus schraubenförmigen Windungen (58) aufgebaut ist, wobei eine Federhärte des zweiten Federelements (52) durch eine Vorspannung des zweiten Federelements (52) erhöht ist, wodurch die Flexibilität des Schaftkörpers (40) in dem zweiten Abschnitt (44) verringert ist, wobei die Vorspannung durch eine axiale Stauchung der Windungen (58) des zweiten Elements relativ zueinander aufgebracht ist, **dadurch gekennzeichnet, dass** die Vorspannung permanent ist und durch ein flexibles Zugelement (60) aufgebracht wird, das das zweite Federelement (52) axial zusammenzieht, wobei das Zugelement (60) an zwei axial beabstandeten Stellen (64, 66) an dem zweiten Federelement (52) befestigt ist, deren Abstand der Länge des zweiten Federelements (52) im komprimierten Zustand entspricht.

2. Schaft nach Anspruch 1, **dadurch gekennzeichnet, dass** das Zugelement (60) ein zumindest das zweite Federelement (52) umgebendes netzartiges Hüllelement (62) ist.

3. Schaft nach Anspruch 2, **dadurch gekennzeichnet, dass** sich das Hüllelement (62) im Wesentlichen über die gesamte Länge des Schaftkörpers (40) erstreckt.

4. Schaft nach Anspruch 2 oder 3, **dadurch gekennzeichnet, dass** das Hüllelement (62) von einem Außenmantel (70) umgeben ist, der mit dem Hüllelement (62) fest verbunden ist.

5. Schaft nach einem der Ansprüche 1 bis 4, **dadurch gekennzeichnet, dass** das erste Federelement (50) und das zumindest zweite Federelement (52) einstückig miteinander ausgebildet sind.

6. Schaft nach einem der Ansprüche 1 bis 5, **dadurch gekennzeichnet, dass** der Schaftkörper (40) zumindest einen dritten Abschnitt (46) mit einem dritten Flexibilitätsgrad aufweist, wobei der Schaftkörper (40) in dem dritten Abschnitt (46) ein drittes Federelement (48) aufweist, wobei der dritte Flexibilitätsgrad geringer als der erste Flexibilitätsgrad und geringer als der zweite Flexibilitätsgrad ist.

7. Schaft nach Anspruch 6, **dadurch gekennzeichnet, dass** das erste, zweite und zumindest dritte Federelement (48, 50, 52) einstückig miteinander ausgebildet sind.

8. Schaft nach einem der Ansprüche 1 bis 7, **dadurch gekennzeichnet, dass** erste Federelement (50) und das zweite Federelement (52) im nicht vorgespannten Zustand des zweiten Federelements (52) die gleiche Federhärte aufweisen.

9. Schaft nach einem der Ansprüche 1 bis 8, **dadurch gekennzeichnet, dass** das erste Federelement (50) und das zweite Federelement (52) Abschnitte ein und desselben einteiligen Federelements sind.

10. Endoskop, **gekennzeichnet durch** einen flexiblen Schaft (12) nach einem der Ansprüche 1 bis 9.

## Claims

1. A flexible shaft for an endoscope (10), comprising a shaft body (40) which, in the longitudinal direction of the shaft (12), has a first portion (42) with a first degree of flexibility, and, proximally from the first portion (42), has at least a second portion (44) with a second degree of flexibility which is less than the first degree of flexibility, the shaft body (40) having, in the first portion (42), at least a first spring element (50), and, in the second portion (44), at least a second spring element (52), at least the second spring element (52) being constructed from helical windings (58), wherein a spring hardness of the second spring element (52) is increased by a pretensioning of the second spring element (52), as a result of which the flexibility of the shaft body (40) is reduced in the second portion (44), wherein the pretensioning is applied by an axial compression of the windings (58) relative to one another, **characterized in that** the pretensioning is permanent and is applied by a flexible traction element (60) which pulls the second spring element (52) axially together, the traction element (60) being attached to the second spring element (52) at two sites (64, 66) situated axially apart from one another, the distance between them corresponding to the length of the second spring element (52) in the compressed state.

2. The shaft of claim 1, **characterized in that** the traction element (60) is a net-like sleeve element (62) surrounding the second spring element (52).

3. The shaft of claim 2, **characterized in that** the sleeve element (62) extends substantially along the entire length of the shaft body (40).

4. The shaft of claim 2 or 3, **characterized in that** the sleeve element (62) is surrounded by an outer jacket (70) which is firmly connected to the sleeve element (62).

5. The shaft of anyone of claims 1 through 4, **characterized in that** the first spring element (50) and the at least second spring element (52) are formed integrally with one another.

6. The shaft of anyone of claims 1 through 5, **characterized in that** the shaft body (40) has at least a third portion (46) with a third degree of flexibility, the shaft body (40) having a third spring element (48) in the third portion (46), the third degree of flexibility being less than the first degree of flexibility and less than the second degree of flexibility.

7. The shaft of claim 6, **characterized in that** the first, second and at least third spring element (48, 50, 52) are formed integrally with one another.

8. The shaft of anyone of claims 1 through 7, **characterized in that** the first spring element (50) and the second spring element (52) have the same spring hardness when the second spring element (52) is in the non-pretensioned state.

9. The shaft of anyone of claims 1 through 8, **characterized in that** the first spring element (50) and the second spring element (52) are portions of one and the same one-piece spring element.

10. An endoscope, **characterized by** a flexible shaft (12) according to anyone of claims 1 through 9.

## Revendications

1. Tige flexible pour un endoscope (10), avec un corps de tige (40) qui comprend dans le sens longitudinal de la tige (12) une première section (42) avec un premier degré de flexibilité et située de façon proximale par rapport à la première section (42) au moins une deuxième section (44) avec un deuxième degré de flexibilité, qui est inférieur au premier degré de flexibilité, le corps de la tige comprenant dans la première section (42) au moins un premier élément ressort (50) et dans la deuxième section (44) au moins un deuxième élément ressort (52), au moins le deuxième élément ressort (52) étant constitué de spires hélicoïdales (58), la dureté de ressort du deuxième élément ressort (52) étant accrue par une précontrainte du deuxième élément ressort (52), par le biais de laquelle la flexibilité du corps de tige (40) est réduite dans la deuxième section (44), la précontrainte étant exercée par une compression axiale des spires (58) du deuxième élément les unes par rapport aux autres, **caractérisée en ce que** la précontrainte est permanente et appliquée par un élément de traction flexible (60) qui rétracte de façon axiale le deuxième élément ressort (52), l'élément de traction (60) étant fixé en deux points (64, 66) espacés de façon axiale sur le deuxième élément ressort (52), lequel écart correspond à la longueur du deuxième élément ressort (52) à l'état comprimé.

2. Tige selon la revendication 1, **caractérisée en ce que** l'élément de traction (60) est un élément enveloppant (62) sous forme de maillage entourant le deuxième élément ressort (52).

3. Tige selon la revendication 2, **caractérisée en ce que** l'élément enveloppant (62) s'étire essentiellement sur la longueur du corps de tige (40).

4. Tige selon la revendication 2 ou 3, **caractérisée en ce que** l'élément enveloppant (62) est entouré d'une enveloppe extérieure (70) qui est solidement reliée à l'élément enveloppant (62).

5. Tige selon l'une des revendications 1 à 4, **caractérisée en ce que** le premier élément ressort (50) et le au moins deuxième élément ressort (52) sont conçus ensemble d'un seul tenant.

6. Tige selon l'une des revendications 1 à 5, **caractérisée en ce que** le corps de tige comprend au moins une troisième section (46) avec un troisième degré de flexibilité, le corps de tige (40) comprenant dans la troisième section (46) un troisième élément ressort (48), le troisième degré de flexibilité étant inférieur au premier degré de flexibilité et inférieur au deuxième degré de flexibilité.

7. Tige selon la revendication 6, **caractérisée en ce que** le premier, le deuxième et au moins le troisième élément ressort (48, 50, 52) sont formés ensemble d'un seul tenant.

8. Tige selon l'une des revendications là 7, **caractérisée en ce que** le premier élément ressort (50) et le deuxième élément ressort présentent la même dureté de ressort lorsque le deuxième élément ressort (52) est en état non précontraint.

9. Tige selon l'une des revendications là 8, **caractérisée en ce que** le premier élément de ressort (50) et le deuxième élément ressort (52) sont des sections d'un seul et même élément ressort d'un seul tenant.

10. Endoscope **caractérisé par** une tige flexible (12) selon l'une des revendications 1 à 9.
